# EUROPÄISCHE PATENTSCHRIFT

(11) **EP 0 335 221 B1**
(45) Veröffentlichungstag und Bekanntmachung des Hinweises auf die Patenterteilung: **29.12.1993**
(21) Anmeldenummer: 89104991.8
(22) Anmeldetag: 21.03.1989
(51) Int. Cl.: C07C 45/85, C07C 45/82

(54) **Verfahren zur Gewinnung von 2-Methylalkanalen aus Gemischen isomerer Aldehyde**
Method for obtaining 2-methylalkanals from mixtures of isomeric aldehydes
Procédé pour obtenir les alkanales-2-méthyles des mélanges d'aldéhydes isomères

(30) Priorität: 31.03.1988 DE 3811039
(43) Veröffentlichungstag der Anmeldung: 04.10.1989
(73) Patentinhaber: HOECHST AKTIENGESELLSCHAFT, 65926 Frankfurt am Main (DE)
(72) Erfinder: Weber, Jürgen, Dr. Dipl.-Chem., D-4200 Oberhausen 11 (DE); Lappe, Peter, Dr. Dipl.-Chem., D-4220 Dinslaken (DE); Springer, Helmut, Dipl.-Ing., D-4200 Oberhausen 11 (DE)

(56) Entgegenhaltungen:
- EP-B- 0 322 660
- DE-A- 2 218 305
- GB-A- 2 078 748

## Beschreibung

Die vorliegende Erfindung betrifft ein Verfahren zur Gewinnung von 2-Methylalkanalen ausgenommen 2-Methylbutanal aus den Gemischen isomerer Aldehyde, die durch Hydroformylierung von Gemischen isomerer Olefine erhalten werden.

2-Methylalkanale sind wertvolle Zwischenprodukte für zahlreiche technisch ausgeübte Verfahren. Sie werden unter anderem durch Oxidation zu 2-Methylalkancarbonsäuren weiterverarbeitet, die z.B. als Riechstoff-Vorprodukte Verwendung finden. 2-Methylalkanale werden weiterhin als Rohstoffe zur Herstellung umfangreich verwendeter Pharmazeutika und Pflanzenschutzmittel eingesetzt.

Das technisch und wirtschaftlich wichtigste Verfahren zur Herstellung von Aldehyden mit mehr als 2 Kohlenstoffatomen ist die Hydroformylierung, d.h. die Anlagerung von Kohlenmonoxid und Wasserstoff an Olefine unter der Einwirkung von Metallcarbonylverbindungen als Katalysatoren. Von den zahlreichen untersuchten Metallen haben lediglich Kobalt und Rhodium und deren Verbindungen als Katalysatoren Bedeutung erlangt. Die Umsetzung führt im allgemeinen zu einem Gemisch isomerer Aldehyde. So entstehen aus endständigen Olefinen n-Aldehyde und daneben die isomeren alpha-Methylaldehyde. Lediglich bei symmetrischen oder sterisch gehinderten Olefinmolekülen erhält man einheitliche Produkte.

Durch Auswahl geeigneter Katalysatoren und Einhaltung entsprechender Reaktionsbedingungen läßt sich das Verhältnis von n- und iso-Verbindung im Reaktionsprodukt in weiten Bereichen variieren.

Die Vielfalt der Aldehyde im Hydroformylierungsprodukt wird noch dadurch erhöht, daß man häufig nicht von einheitlichen Olefinen ausgeht, sondern von den bei der Erdölverarbeitung erhaltenen Gemischen isomerer Olefine. Solche Gemische bestehen aus Olefinen mit der gleichen Anzahl Kohlenstoffatome aber unterschiedlicher Struktur.

So erhält man bei der Hydroformylierung von Gemischen aus 2-Methylbuten-1 und 2-Methylbuten-2 nebeneinander 2,3-Dimethylbutanal, 3-Methylpentanal und 4-Methylpentanal. Gemische aus 3-Methyl-2-penten und 2-Ethylbuten-1 ergeben als Hydroformylierungsprodukt 2,3-Dimethylpentanal, 3-Ethylpentanal und 4-Methylhexanal.

Die Trennung von Aldehydgemischen erfolgt in technischen Prozessen überwiegend durch fraktionierte Destillation. Wegen der Oxidationsempfindlichkeit der meisten Aldehyde, ihrer Neigung zu höhermolekularen Folgeprodukten zu kondensieren und sich thermisch zu zersetzen, sind bestimmte Vorsichtsmaßnahmen bei der Destillation einzuhalten. Empfindliche Aldehyde werden bei möglichst milden Temperaturen und nach vollständiger Entfernung der Katalysatoren destillativ getrennt. Darüber hinaus wendet man dei Verfahren der azeotropen und der extraktiven Destillation an, um Kondensations- und Zersetzungsreaktionen zu vermeiden.

Die Komponenten des bei der Hydroformylierung von einheitlichen Olefinen oder von Gemischen aus Olefinen gleicher C-Zahl erhaltenen Aldehydgemisches weisen nur wenig unterschiedliche Siedepunkte auf. Eine destillative Trennung ist daher nur mit äußerst trennstarken Kolonnen bei hohen Rücklaufverhältnissen möglich. Diese Bedingungen stehen nicht nur der Wirtschaftlichkeit des Trennverfahrens entgegen. Es können auch erhebliche Ausbeuteminderungen auftreten, verursacht durch die Bildung höhersiedender Verbindungen aus den thermisch wenig stabilen n-Aldehyden.

Da die vorstehend geschilderten Probleme allgemein auftreten, hat man sich bemüht, Verfahren zu entwickeln, die es erlauben, Aldehydgemische, wie sie z.B. bei der Hydroformylierung gebildet werden, einfach und effektiv zu trennen.

Nach der DE 28 33 538 C2 werden alpha-methylverzweigte Aldehyde der allgemeinen Formel R-CH(CH₃)-CHO, wobei R für geradkettige Alkylreste mit 7 bis 12 Kohlenstoffatomen steht, von den geradkettigen Verbindungen mit der gleichen Anzahl Kohlenstoffatome durch thermische Behandlung, die in einer Destillationskolonne erfolgen kann, abgetrennt. Hierbei destillieren die verzweigten Aldehyde ab, während die geradkettigen Aldehyde in schwerflüchtige Verbindungen übergehen und im Sumpf der Kolonne zurückbleiben. Dieses Verfahren wird bevorzugt dann angewendet, wenn die geradkettigen Aldehyde nicht verwertet werden können.

Gegenstand der DE 24 59 152 C2 ist ein Verfahren zur Gewinnung reiner n-Aldehyde aus Mischungen mit ihren Isomeren, insbesondere aus dem rohen, bei der Hydroformylierung endständiger Olefine anfallenden und gegebenenfalls vom Katalysator abgetrennten Reaktionsgemisch. Es besteht darin, daß das Reaktionsgemisch in Gegenwart eines Lösungsmittels mit der zur Fällung des n-Aldehyds als Hydrogensulfit-Additionsverbindung erforderlichen, stöchiometrischen oder unterstöchiometrischen Menge Alkalihydrogensulfit umgesetzt, der entstehende Niederschlag abgetrennt, gewaschen und anschließend in üblicher Weise gespalten wird. Wegen des nicht unerheblichen Chemikalienaufwandes eignet sich dieses Verfahren insbesondere zur Gewinnung wertvoller n-Aldehyde.

Ebenfalls über die Bisulfitverbindungen erfolgt die Zerlegung von Gemischen isomerer Aldehyde nach einer Arbeitsweise, die in der DE 960 187 Cl beschrieben ist. Das Aldehydgemisch wird zunächst nach Behandeln mit der wäßrigen Lösung eines neutralen Sulfits und einer etwa äquivalenten Menge einer schwach sauren Verbindung in ein Gemisch der Bisulfitverbindungen überführt, das man sodann auf steigende Temperaturen erhitzt. Die hierbei in der Reihenfolge ihrer Siedepunkte nacheinander freiwerdenden Aldehyde zieht man getrennt ab und verwendet die zurückbleibende, abgekühlte Lösung erneut für die Herstellung der Bisulfitverbindungen. Dieser Prozeß ist nur auf die Trennung der leicht flüchtigen niederen Aldehyde anwendbar, da höhere Aldehyde sich bei ihren Siedepunkten schon merklich zersetzen und daher destillativ nicht abgetrennt werden können.

Ein Verfahren zur Reindarstellung von n-Aldehyden aus n/iso-Aldehydgemischen besteht in der Behandlung der Gemische mit nicht-oxidierenden starken Mineralsäuren (DE 22 18 305). Die geradkettigen Aldehyde gehen dabei in 1,3,5-Trioxane über, die durch fraktionierte Kristallisation abgetrennt werden. Anschließend depolymerisiert man die Trioxane in einer Destillationsapparatur unter Zusatz geringer Mengen Phosphor(V)-oxid. Das Verfahren beruht also auf der unterschiedlichen Kristallisationsfähigkeit und Löslichkeit der trimeren n-Alkanale und iso-Alkanale. Es ist nicht anwendbar, wenn außer den n-Alkanalen auch die iso-Alkanale kristallin sind. Die Bedeutung dieser Arbeitsweise ist daher begrenzt.

Nach der Lehre der EP-B-0 322 660 gewinnt man 2-Methylbutanal aus den Gemischen der isomeren C₅-Aldehyde durch Destillation des Aldehydgemischs in Gegenwart von Formaldehyd und einem Aldolisierungskatalystor.

Es bestand somit die Aufgabe, ein Verfahren zu entwickeln, das es erlaubt, 2-Methylalkanale aus dem Gemisch isomerer Aldehyde, die bei der Hydroformylierung isomerer Olefine anfallen, zu gewinnen.

Überraschenderweise wird diese Aufgabe gelöst durch ein Verfahren zur Gewinnung von 2-Methylalkanalen ausgenommen 2-Methylbutanal aus den Gemischen der isomeren Aldehyde, die bei der Hydroformylierung isomerer Olefine entstehen. Es ist dadurch gekennzeichnet, daß man das Aldehydgemisch in Gegenwart von Formaldehyd und einem Aldolisierungskatalysator destilliert.

Es ist bekannt, daß Aldehyde, die in alpha-Stellung zur Carbonylgruppe eine Methylengruppe aufweisen, mit Formaldehyd in Gegenwart geeigneter Katalysatoren zu 2-Methylenaldehyden reagieren. Die Umsetzung erfolgt üblicherweise unter Druck und - abhängig von der gewählten Temperatur - bei Verweilzeiten, die in der Größenordnung von wenigen Sekunden bis zu Stunden liegen.

Ähnlich reaktiv sind auch Aldehyde, die an alpha-ständigen Kohlenstoffatomen nur ein Wasserstoffatom tragen. Sie ergeben mit Formaldehyd sehr leicht die entsprechenden alpha-Methylolverbindungen.

Es war daher nicht vorauszusehen, daß es trotz der hohen Reaktivität sowohl der in alpha-Stellung unverzweigten als auch der in alpha-Stellung monoverzweigten Aldehyde möglich ist, ein Gemisch beider Isomerenarten durch gezielte Umsetzung der in alpha-Stellung unverzweigten Bestandteile in die Komponenten zu zerlegen.

Das neue Verfahren eignet sich ganz allgemein zur Gewinnung von 2-Methylalkanalen ausgenommen 2-Methylbutanal aus den Gemischen isomerer Aldehyde, die bei der Hydroformylierung von Olefinisomeren anfallen. Unter den Begriffen Olefinisomere oder isomere Olefine werden Verbindungen verstanden, die sich durch die Anordnung der Atome oder die Lage der Doppelbindung im Molekül unterscheiden.

Typische Vertreter solcher Olefinisomeren sind z.B. die Produkte der Dehydratisierung von 2-Methylbutanol oder 2-Ethylbutanol. Je nach Herstellungs- und Aufarbeitungsbedingungen sind sie etwa wie folgt zusammengesetzt:

| 2-Methylbuten-1/2-Gemische | |
|---|---|
| Komponente | Gew.-% |
| 3-Methylbuten-1 | 0,5 - 4 % |
| Pentene | 0,5 - 5 % |
| 2-Methylbuten-1 | 50 - 85 % |
| 2-Methylbuten-2 | 15 - 30 % |
| Sonstige | 0,5 - 5 % |

| 3-Methyl-2-penten-/2-Ethylbuten-1-Gemische | |
|---|---|
| Komponente | Gew.-% |
| C₄-C₅-Olefine | 0,5 - 2 % |
| Hexene | 0,5 - 4 % |
| 2-Ethylbuten-1 | 5 - 10 % |
| trans-3-Methyl-2-penten | 20 - 40 % |
| cis-3-Methyl-2-penten | 40 - 60 % |

Die vorstehend beschriebenen Gemische sind nur beispielhaft aufgeführt.

Selbstverständlich können auch andere Olefingemische hydroformyliert und zur Gewinnung von 2-Methylalkanalen nach dem neuen Verfahren verwendet werden.

Die Hydroformylierung von Olefinen gehört zum Stand der Technik. Sie kann nach verschiedenen Varianten unter Einsatz von Kobalt- oder Rhodiumkatalysatoren erfolgen. Eine umfassende Darstellung der Reaktion findet sich z.B. in der Monographie New Syntheses with Carbon Monoxide, Hrsg. J. Falbe, Springer-Verlag 1980.

Hydroformyliert man die oben aufgeführten Olefingemische z.B. mit Rhodium als Katalysator bei 90 bis 140°C und 20 bis 30 MPa, so erhält man nach Abtrennung des Katalysators durch Flashdestillation an einem Dünnschichtverdampfer Aldehydgemische etwa folgender Zusammensetzung:

| Aldehydgemisch auf Basis 2-Methylbuten-1/2 | |
|---|---|
| 2,3-Dimethylbutanal | 55 - 80 % |
| 3-Methylpentanal | 10 - 30 % |
| 4-Methylpentanal | 3 - 12 % |
| Sonstige | 1 - 4 % |

| Aldehydgemisch auf Basis 3-Methyl-2-penten/2-Ethylbuten-1 | |
|---|---|
| 2,3-Dimethylpentanal | 55 - 90 % |
| 3-Ethylpentanal | 5 - 15 % |
| 4-Methylhexanal | 5 - 15 % |
| Sonstige | 0,5 - 2 % |

Zur Umsetzung des Aldehydgemisches mit Formaldehyd wendet man je Mol in alpha-Stellung unverzweigten Aldehyd 1 bis 2 und insbesondere 1,1 bis 1,4 Mol Formaldehyd an. Der Formaldehyd wird bevorzugt als Lösung in Wasser eingesetzt, geeignet sind aber auch Lösungen in Alkoholen oder polymerisierter Formaldehyd, z.B. Paraformaldehyd. Im allgemeinen wird dem Reaktionsgemisch die gesamte Menge Formaldehyd zu Beginn der Umsetzung zugegeben. Es kann aber auch zweckmäßig sein, ihm anteilsweise dem Aldehydgemisch zuzusetzen.

Die Reaktion der alpha-unverzweigten Aldehyde mit Formaldehyd erfolgt in Gegenwart von Aminen als Katalysatoren. Bewährt haben sich sekundäre Amine der allgemeinen Formel R¹-NH-R², wobei R¹ und R² gleich oder verschieden sind und für Alkylreste mit je 1 bis 12, vorzugsweise 3 bis 5 Kohlenstoffatome stehen. Besonders geeignet ist Di-n-butylamin. Vorteilhaft führt man die Reaktion in Gegenwart von Monocarbonsäuren mit 1 bis 10 Kohlenstoffatomen oder Di- bzw. Polycarbonsäuren mit 2 bis 10 Kohlenstoffatomen durch. Bevorzugt werden Monocarbonsäuren mit 3 bis 5 Kohlenstoffatomen. Als Di- und Polycarbonsäuren kommen aromatische, araliphatische und vorzugsweise aliphatische Verbindungen in Betracht. Geeignet sind z.B. Essigsäure, Propionsäure, n-Buttersäure, iso-Buttersäure, Oxalsäure, Bernsteinsäure, Weinsäure. Bevorzugt setzt man n-Buttersäure ein. Je Mol des alpha-unverzweigten Aldehyds wendet man 0,025 bis 0,3, vorteilhaft 0,1 bis 0,2 Mol Amin an. Je Mol alpha-unverzweigten Aldehyds werden 0,05 bis 0,5 und insbesondere 0,15 bis 0,3 Äquivalente Carbonsäuren eingesetzt.

Die Reaktion der alpha-unverzweigten Aldehyde mit Formaldehyd führt zur Bildung von alpha-Methylolaldehyden, die im allgemeinen unbeständig sind und unter Wasserabspaltung in die entsprechenden alpha-Methylenaldehyde (alpha-Alkylacroleine) übergehen. Der Siedepunkt dieser alpha-Methylenaldehyde unterscheidet sich deutlich von dem des ein Kohlenstoffatom weniger enthaltenden alpha-Methylaldehyds. Daher ist es ohne Schwierigkeiten möglich, die Aldehyde durch Destillation voneinander zu trennen.

Für das erfindungsgemäße Verfahren ist es charakteristisch, die Umsetzung des Aldehydgemisches mit Formaldehyd während der Destillation vorzunehmen. Zum Einsatz gelangen handelsübliche Destillationskolonnen mit Glasringen oder Metallwendeln als Füllkörper. Die Anzahl der theoretischen Böden beträgt in der Regel 9 bis 72 und bevorzugt 24 bis 36. Das Rücklaufverhältnis ist von der Art der eingesetzten Kolonne abhängig. Es kann z.B. bei einer Füllkörperkolonne mit 24 theoretischen Böden auf 5 Teile Rücklauf je Teil abgenommenes Kopfprodukt eingestellt werden. Ebenso ist die Temperatur des Sumpfproduktes von der Kolonne abhängig.

Nach dem neuen Verfahren erhält man 2-Methylalkanale in einer Reinheit von >97 %, sie sind lediglich durch geringe Anteile alpha-unverzweigte Aldehyde sowie inerte Bestandteile verunreinigt. Die Inerten stören bei der Weiterverarbeitung der 2-Methylalkanale z.B. zu Säure, Amin, Alkohol oder Diol nicht und sind aus dem Folgeprodukt des Aldehyds leicht zu entfernen.

In den folgenden Beispielen wird die Erfindung näher erläutert. Sie ist aber selbstverständlich nicht auf die hier beschriebenen Ausführungsformen beschränkt.

### Beispiel 1: Herstellung von 2,3-Dimethylbutanal

Bei der Hydroformylierung von 2-Methyl-1-buten und nachfolgender destillativer Aufarbeitung des Aldehydgemisches erhält man eine Hauptfraktion der Zusammensetzung:

| | |
|---|---|
| Vorlauf | 1,01 Gew.-% |
| 2,3-Dimethylbutanal | 69,57 Gew.-% |
| 3-Methylpentanal | 21,13 Gew.-% |
| 4-Methylpentanal | 7,46 Gew.-% |
| Nachlauf | 0,83 Gew.-% |

2000 g dieses Aldehydgemisches (5,71 mol alpha unverzweigte Aldehyde) werden mit 812,9 g Formalin (29,5 Gew.-% Formaldehyd, entsprechend 7,99 mol), 110,5 g Di-n-butylamin (0,86 mol) und 150,9 g n-Buttersäure (1,71 mol) versetzt und unter Rühren bei 0,1 MPa fraktioniert destilliert. Die verwendete Füllkörperkolonne hat 24 theoretische Böden. Das Rücklaufverhältnis wird auf 5 Teile Rücklauf je Teil abgenommenes Kopfprodukt eingestellt. Die nachfolgende Tabelle enthält die Kopf- und Sumpftemperaturen der einzelnen Fraktionen sowie die zugehörigen Auswaagen.

| Fraktion | max.Temp. des Kopfproduktes (°C) | max.Temp. des Sumpfproduktes (°C) | Auswaage org.Phase (g) | Auswaage wäßr.Phase (g) |
|---|---|---|---|---|
| 1 | 85 | 90 | 84,1 | 24,7 |
| 2 | 87 | 96 | 1144,2 | 346,2 |
| 3 | 90 | 98 | 156,2 | 59,7 |
| 4 | 92 | 144 | 235,8 | 106,5 |
| 5 | 104 | 220 | 326,7 | 220,4 |
| Rückstand | | | 369,8 | |

Nach den gaschromatographischen Analysen setzten sich die organischen Phasen wie folgt zusammen:

| | Zusammensetzung der org. Phase (GC-Analyse) (%) | | | | |
|---|---|---|---|---|---|
| | 1.Fraktion | 2.Fraktion | 3.Fraktion | 4.Fraktion | 5.Fraktion |
| Vorlauf | 11,65 | 0,85 | 0,86 | 5,38 | 4,43 |
| 2,3-Dimethylbutanal | 79,22 | 97,14 | 49,50 | 27,25 | 6,51 |
| 3-Methylpentanal | 0,09 | 0,01 | 0,02 | 0,08 | 0,10 |
| 4-Methylpentanal | 7,04 | 1,24 | 0,01 | 0,01 | - |
| alpha-Alkylacroleine | 0,03 | 0,46 | 45,83 | 64,22 | 52,76 |
| Nachlauf | 1,97 | 0,50 | 3,78 | 3,06 | 36,20 |

Demzufolge werden 79,9 % des eingesetzten 2,3-Dimethylbutanals in der organischen Phase der 2. Fraktion in weitgehend isomerenfreier Form gewonnen.

### Beispiel 2: Herstellung von 2,3-Dimethylpentanal

Bei der Hydroformylierung von 3-Methyl-2-penten und nachfolgender destillativer Aufarbeitung des Aldehydgemisches erhält man eine Hauptfraktion der Zusammensetzung:

| | |
|---|---|
| Vorlauf-Komponente | 0,52 Gew.-% |
| 2,3-Dimethylpentanal | 77,61 Gew.-% |
| 3-Ethylpentanal | 10,89 Gew.-% |
| 4-Methylhexanal | 10,72 Gew.-% |
| Nachlauf-Komponenten | 0,26 Gew.-% |

2000 g dieses Aldehydgemisches (3,78 mol alpha-unverzweigte Aldehyde) werden mit 538,2 g Formalin (29,5 Gew.-% Formaldehyd, entsprechend 5,29 mol), 73,1 g Di-n-butylamin (0,57 mol) und 99,9 g n-Buttersäure (1,13 mol) versetzt und unter Rühren bei 0,1 MPa fraktioniert destilliert. Die verwendete Füllkörperkolonne hat 24 theoretische Böden. Das Rücklaufverhältnis wird auf 5 Teile Rücklauf je Teil abgenommenes Kopfprodukt eingestellt. Die nachfolgende Tabelle enthält die Kopf- und Sumpftemperaturen der einzelnen Fraktionen sowie die zugehörigen Auswaagen:

| Fraktion | max.Temp. des Kopfproduktes (°C) | max.Temp. des Sumpfproduktes (°C) | Auswaage org.Phase (g) | Auswaage wäßr.Phase (g) |
|---|---|---|---|---|
| 1 | 140 | 180 | 1421,5 | 495,1 |
| 2 | 147 | 193 | 104,1 | - |
| 3 | 148 | 208 | 95,9 | - |
| 4 | 152 | 240 | 172,2 | - |
| Rückstand | | | 422,4 | |

Nach den gaschromatographischen Analysen setzten sich die organischen Phasen wie folgt zusammen:

| | Zusammensetzung der org. Phasen (GC-Analyse) (%) | | | |
|---|---|---|---|---|
| | 1.Fraktion | 2.Fraktion | 3.Fraktion | 4.Fraktion |
| Vorlauf | 1,71 | 1,61 | 1,33 | 2,16 |
| 2,3-Dimethylpentanal | 97,68 | 76,71 | 13,14 | 4,62 |
| 3-Ethylpentanal | 0,01 | 0,11 | 0,15 | 0,11 |
| 4-Methylhexanal | 0,03 | 0,19 | 0,78 | 1,60 |
| alpha-Alkylacroleine | 0,08 | 17,48 | 77,84 | 81,04 |
| Nachlauf | 0,49 | 3,90 | 6,76 | 10,47 |

Demzufolge werden 89,5 % des eingesetzten 2,3-Dimethylpentanals in der organischen Phase der 1. Fraktion in nahezu isomerenfreier Form gewonnen.

## Patentansprüche

1. Verfahren zur Gewinnung von 2-Methylalkanalen, ausgenommen 2-Methylbutanal, aus den Gemischen der isomeren Aldehyde, die bei der Hydroformylierung isomerer Olefine entstehen, dadurch gekennzeichnet, daß man das Aldehydgemisch in Gegenwart von Formaldehyd und einem Aldolisierungskatalysator destilliert.

2. Verfahren nach Anspruch 1, dadurch gekennzeichnet, daß man je Mol im Aldehydgemisch enthaltener in alpha-Stellung unverzweigter Aldehyde 1 bis 2, insbesondere 1,1 bis 1,4 Mol Formaldehyd anwendet.

3. Verfahren nach Anspruch 2, dadurch gekennzeichnet, daß der Formaldehyd als Lösung in Wasser angewendet wird.

4. Verfahren nach einem oder mehreren der Ansprüche 1 bis 3, dadurch gekennzeichnet, daß man als Aldolisierungskatalysator sekundäre Amine der allgemeinen Formel R¹-NH-R² einsetzt, wobei R¹ und R² gleich oder verschieden sind und für Alkylreste mit je 1 bis 12, vorzugsweise 3 bis 5 Kohlenstoffatome stehen.

5. Verfahren nach Anspruch 4, dadurch gekennzeichnet, daß das sekundäre Amin Di-n-butylamin ist.

6. Verfahren nach einem oder mehreren der Ansprüche 1 bis 5, dadurch gekennzeichnet, daß man die Reaktion in Gegenwart einer Monocarbonsäure mit 1 bis 10 Kohlenstoffatomen oder Di- bzw. Polycarbonsäuren mit 2 bis 10 Kohlenstoffatomen durchführt.

7. Verfahren nach Anspruch 6, dadurch gekennzeichnet, daß man die Reaktion in Gegenwart einer Monocarbonsäure mit 3 bis 5 Kohlenstoffatomen, insbesondere n-Buttersäure, durchführt.

8. Verfahren nach einem oder mehreren der Ansprüche 1 bis 7, dadurch gekennzeichnet, daß man je Mol in alpha-Stellung unverzweigter Aldehyde 0,025 bis 0,3, insbesondere 0,1 bis 0,2 Mol Amin anwendet.

9. Verfahren nach einem oder mehreren der Ansprüche 1 bis 8, dadurch gekennzeichnet, daß man je Mol in alpha-Stellung unverzweigter Aldehyde 0,05 bis 0,5, insbesondere 0,15 bis 0,3 Äquivalente Carbonsäuren einsetzt.

## Claims

1. A process for the recovery of 2-methylalkanals, except 2-methylbutanal, from mixtures of isomeric aldehydes, which are formed during the hydroformylation of isomeric olefins, characterised in that the aldehyde mixture is distilled in the presence of formaldehyde and an aldolisation catalyst.

2. A process according to claim 1, characterised in that 1 to 2, in particular 1.1 to 1.4 moles of formaldehyde are used per mole of aldehydes unbranched in the alpha position and contained in the aldehyde mixture.

3. A process according to claim 2, characterised in that the formaldehyde is used as a solution in water.

4. A process according to one or more of the claims 1 to 3, characterised in that secondary amines of the general formular R¹-NH-R² are used as aldolisation catalysts, where R¹ and R² are the same or different and denote alkyl groups each with 1 to 12, preferably 3 to 5 carbon atoms.

5. A process according to claim 4, characterised in that the secondary amine is di-n-butylamine.

6. A process according to one or more of the claims 1 to 5, characterised in that the reaction is performed in the presence of a monocarboxylic acid with 1 to 10 carbon atoms or dicarboxylic or polycarboxylic acids with 2 to 10 carbon atoms.

7. A process according to claim 6, characterised in that the reaction is performed in the presence of a monocarboxylic acid with 3 to 5 carbon atoms, in particular n-butyric acid.

8. A process according to one or more of the claims 1 to 7, characterised in that 0.025 to 0.3, in particular 0.1 to 0.2 moles of amine are used per mole of aldehydes unbranched in the alpha position.

9. A process according to one or more of the claims 1 to 8, characterised in that 0.05 to 0.5, in particular 0.15 to 0.3 equivalents of carboxylic acids are used per mole of aldehydes unbranched in the alpha position.

## Revendications

1. Procédé pour isoler les 2-méthylalcanals, à l'exception du 2-méthylbutanal, des mélanges d'aldéhydes isomères formés à l'hydroformylation d'oléfines isomères, caractérisé en ce que l'on distille le mélange d'aldéhydes en présence de formaldéhyde et d'un catalyseur d'aldolisation.

2. Procédé selon revendication 1, caractérisé en ce que l'on utilise de 1 à 2, plus spécialement de 1,1 à 1,4 mol de formaldéhyde par mole d'aldéhydes non ramifiés en position α contenus dans le mélange d'aldéhydes.

3. Procédé selon revendication 2, caractérisé en ce que le formaldéhyde est mis en oeuvre à l'état de solution dans l'eau.

4. Procédé selon une ou plusieurs des revendications 1 à 3, caractérisé en ce que l'on utilise en tant que catalyseurs d'aldolisation des amines secondaires de formule générale R¹-NH-R² dans laqeulle R¹ et R², qui peuvent avoir des significations identiques ou différentes, représentent chacun un groupe alkyle en C₁-C₁₂, de préférence en C₃-C₅.

5. Procédé selon revendication 4, caractérisé en ce que la diamine secondaire utilisée est la di-n-butylamine.

6. Procédé selon une ou plusieurs des revendications 1 à 5, caractérisé en ce que l'on effectue la réaction en présence d'un acide monocarhoxylique en C₁-C₁₀ ou d'acides di- ou polycarboxyliques en C₂-C₁₀.

7. Procédé selon revendication 6, caractérisé en ce que l'on effectue la réaction en présence d'un acide monocarboxylique en C₃-C₅, en particulier l'acide n-butyrique.

8. Procédé selon une ou plusieurs des revendications 1 à 7, caractérisé en ce que l'on utilise de 0,025 à 0,3, plus spécialement de 0,1 à 0,2 mol d'amine par mole d'aldéhydes non ramifiés en position α.

9. Procédé selon une ou plusieurs des revendications 1 à 8, caractérisé en ce que l'on utilise de 0,05 à 0,5, plus spécialement de 0,15 à 0,3 équivalent d'acides carboxyliques par mole d'aldéhydes non ramifiés en position α.
